# EUROPEAN PATENT SPECIFICATION

(11) **EP 3 374 335 B1**
(45) Date of publication and mention of the grant of the patent: **28.10.2020**
(21) Application number: 16797616.6
(22) Date of filing: 10.11.2016
(51) Int. Cl.: C07C 5/25, C07C 11/02, C07C 11/107, C07C 15/44, C07C 51/353, C07C 57/12, B01J 27/188, B01J 27/19, B01J 23/28, B01J 23/30, B01J 35/00, B01J 35/10, B01J 37/10, B01J 37/28, B01J 37/00

(54) **PHOTOCATALYTIC PROCESS FOR THE ISOMERISATION OF AN UNSATURATED COMPOUND**
PHOTOKATALYTISCHER VERFAHREN ZUR ISOMERISIERUNG EINER UNGESÄTTIGTEN VERBINDUNG
PROCÉDÉ PHOTOCATALYTIQUE POUR L'ISOMÉRISATION D'UN COMPOSÉ INSATURÉ

(30) Priority: 10.11.2015 GB 201519857
(43) Date of publication of application: 19.09.2018
(73) Proprietor: OXFORD UNIVERSITY INNOVATION LIMITED, Oxford OX2 0JB (GB)
(72) Inventor: XIAO, Tiancun, Headington Oxford OX3 7TL (GB); MA, Wangjing, Beijing 100085 (CN)
(74) Representative: HGF
(86) International application number: PCT/GB2016/053532
(87) International publication number: WO 2017/081471

(56) References cited:
- EP-A1- 0 169 250
- GB-A- 847 066
- US-A1- 2004 147 795
- US-B2- 6 914 029
- US-B2- 8 232 224

## Description

### INTRODUCTION

The present invention relates to catalytic processes for migrating double bonds within unsaturated organic compounds. The present invention also relates to heteropoly acid catalytic compounds useful in the aforementioned catalytic processes.

### BACKGROUND OF THE INVENTION

Isomerization of alkenes is of essential importance for producing high quality transportation fuels. Alkenes with internal C=C double bonds have higher octane number than those with terminal C=C double bonds, leading to an improved combustion efficiency.

Elsewhere, isomerization of alkenes is an important precursor step in the synthesis of branched aldehydes via hydroformylation reactions. Similarly, by migrating C=C double bonds within monomeric starting materials, the extent of branching in the resulting polymer can be controlled to suit the needs of a particular application.

Various studies have explored alkene isomerization using zeolites or other mesoporous material catalysts or catalytic supports (B. Modhera, M. Chakraborty, P.A. Parikh, H.C. Bajaj, Catal. Lett. 132 (2009)168-173*.* G. Shi, J. Shen, Energ. Fuels 23 (2008) 320-326*;* Y. Rao, J. Kang, D. Antonelli, J. Am.Chem. Soc. 130 (2007) 394-395*;* Y. Rao, J. Kang, M. Trudeau, D.M. Antonelli, J. Catal. 266 (2009) 1-8.), or using calcium metal aluminosilicate molecular sieve catalysts (U.S. Pat. No. 3,697,616). Other studies have focused on the isomerization of vinylidene olefins using an iron carbonyl catalyst (U.S. Pat. No. 4,587,374), or the use of non-zeolitic molecular sieves for the isomerization of pentenes (U.S. Pat. No. 5,367,101). GB847,066 describes a process for the isomerization of butylene-1 to butylene-2, comprising contacting butylene-1 with a heteropoly acid at a temperature in the range of from 50 to 300°C and at a liquid hourly space velocity in the range of from 1 to 50 L/L.h.

In spite of the above, there remains a need for alternative means for migrating C=C double bonds within unsaturated organic compounds.

The present invention was devised with the foregoing in mind.

### SUMMARY OF THE INVENTION

According to a first aspect of the present invention there is provided a process for the migration of a carbon-carbon double bond within an unsaturated organic compound having 4 or more carbon atoms, the process comprising the step of:
a) contacting the unsaturated organic compound with a heteropoly acid;
wherein step a) is conducted in the presence of electromagnetic radiation having a wavelength of less than or equal to 700 nm,
and wherein the heteropoly acid is phosphotungstic acid.

Also described herein is a process for the preparation of a heteropoly acid, the process comprising the steps of:
a) admixing:
   i. water,
   ii. a metal source comprising either or both of tungsten and molybdenum,
   iii. a non-metal source comprising one or more of silicon, phosphorus, arsenic and germanium, and
   iv. at least one water-soluble polar organic compound;
b) adjusting the pH of the mixture of step a) to ≤2;
c) isolating the product formed in step b);
d) drying the isolated product; and
e) optionally calcining the dried isolated product.

Also described herein is a heteropoly acid obtainable, obtained or directly obtained by a process defined herein.

Also described herein is a use of a heteropoly acid defined herein in a process for the migration of a C=C as defined herein.

### DETAILED DESCRIPTION OF THE INVENTION

### Catalytic processes

As described hereinbefore, the present invention provides a process for the migration of a carbon-carbon double bond within an unsaturated organic compound having 4 or more carbon atoms, the process comprising the step of:
a) contacting the unsaturated organic compound with a heteropoly acid; wherein step a) is conducted in the presence of electromagnetic radiation having a wavelength of less than or equal to 700 nm,
   and wherein the heteropoly acid is phosphotungstic acid.

When compared with prior art processes, the present invention provides a fast and highly efficient method for the photocatalytic migration of C=C double bonds within unsaturated organic compounds using a catalytic quantity of a phosphotungstic heteropoly acid under visible or UV-vis light.

As used herein, unless otherwise specified the term "unsaturated organic compound" refers to any organic compound comprising at least one C=C double bond. For unsaturated organic compounds comprising more than 4 carbon atoms, more than one C=C double bond may be present, any or all of which may be migrated according to a process defined herein. The unsaturated organic compound may be linear, branched or cyclic (e.g. mono-cyclic, fused bi-, poly-cyclic, or spiro). In addition to a C=C double bond, the unsaturated organic compound may comprises one or more other functional groups. Non-limiting examples of such functional groups include carbonyl, hydroxyl, ether, amino, amido, C=C triple bond, cyano, nitro, halo, aryl, heteroaryl, alkoxy, sulfoxide, sulfonyl, sulfonamide, sulfamoyl, and carbamoyl.

In an embodiment, the unsaturated organic compound has 60 or fewer carbon atoms. Suitably, the unsaturated organic compound has 30 or fewer carbon atoms. More suitably, the unsaturated organic compound has 20 or fewer carbon atoms.

In another embodiment, the C=C double bond of the unsaturated organic compound is a terminal or internal C=C double bond. Terminal C=C double bonds are internalised during the catalytic process of the invention.

In another embodiment, the unsaturated organic compound is a straight chain alkene or a branched alkene or an aryl comprising a straight chain alkene or a branched alkene. Suitably, the unsaturated organic compound comprises only carbon and hydrogen atoms. Exemplary alkenes include hexane, heptene, octane, nonene, decene, dodecene, tetradecene, octadecene and allylbenzene.

In another embodiment, the unsaturated organic compound is a straight chain alkene or a branched alkene comprising only carbon and hydrogen atoms. Suitably, the unsaturated organic compound is a 1-, 2-, 3- or 4- alkene comprising only carbon and hydrogen atoms. Exemplary alkenes include hexane, heptene, octane, nonene, decene, dodecene, tetradecene and octadecene. Suitably, the alkenes include hexane, heptene, octane, nonene and decene.

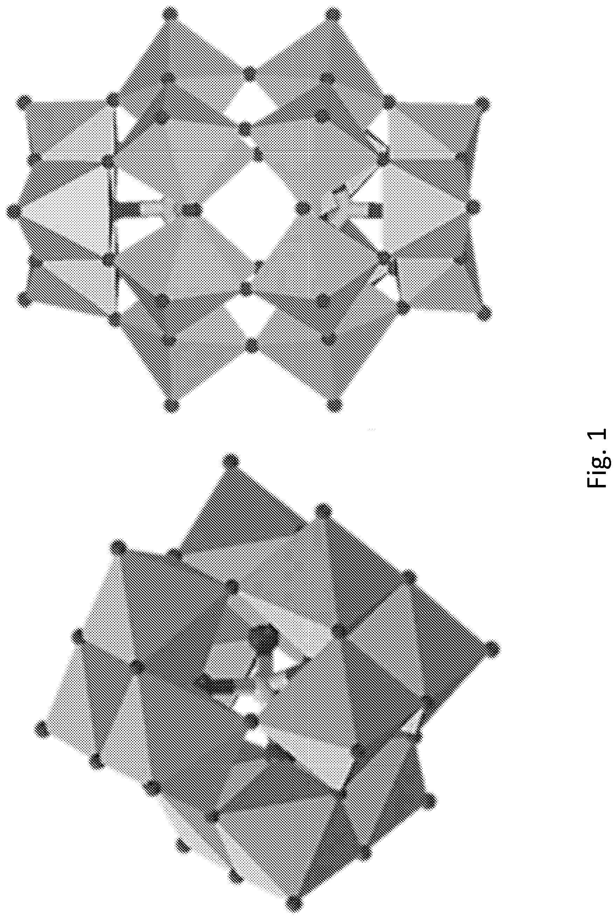

The term "heteropoly acid" will be readily understood by one of skill in the art as being a type of acid formed from a particular combination of hydrogen and oxygen with certain metals and non-metals. Within a heteropoly acid, metal addenda atoms are linked by oxygen atoms to from MO₆ octahedra that cluster together around a central non-metal hetero atom. Exemplary metal addenda atoms include one or more of Mo, W, V, Ru, Rh, Sc, Cr, Mn, Co, Cu, Ni, Zn, Ce, Zr, Nb, Sb and Ti. Exemplary non-metal hetero atoms include one or more of P, Si, As, Ge and B. Heteropoly acids having a variety of structures may be prepared, owing to the numerous possibilities for the manner in which the MO₆ octahedra and central non-metal hetero atoms may cluster together. Two of the more common heteropoly acids are based on the Keggin and Dawson structures, shown in Fig. 1.

In an embodiment, the heteropoly acid has a Keggin or Dawson structure. The Keggin heteropoly anion has the empirical formula, XM₁₂O₄₀ⁿ⁻, wherein X is the non-metal heteroatom (e.g. P⁵⁺, Si⁴⁺ or B³⁺) and M is the metal addenda atom (e.g. Mo, W, V, Ce, Zr, Nb, Sb and Ti). Exemplary Keggin heteropoly acids in which X is phosphorus include H₃PW₁₂O₄₀, H₃PMo₁₂O₄₀, H₄PNbW₁₁O₄₀, H₄PNbMo₁₁O₄₀, H₄PVW₁₁O₄₀, H₄PVMo₁₁O₄₀, H₅PNb₂W₁₀O₄₀, H₅PNb₂MO₁₀O₄₀, H₅PV₂W₁₀O₄₀, H₅PV₂Mo₁₀O₄₀, H₅PV₂WMo₉O₄₀, H₄PVW₂MO₉O₄₀, H₆PV₃W₉O₄₀, H₆PV₃Mo₉O₄₀, H₅PCeW₁₁O₄₀, H₃PW₆Mo₆O₄₀, H₇PV₄W₄O₄₀, H₇PV₄Mo₈O₄₀, H₅PZrW₁₁O₄₀ and H₅PTiW₁₁O₄₀. It will be understood that phosphorus can be substituted for one or more other non-metal hetero atoms discussed herein. The Dawson heteropoly anion has the empirical formula, X₂M₁₈O₆₂ⁿ⁻, wherein X is the non-metal heteroatom and M is the metal addenda atom. Exemplary Dawson heteropoly acids in which X is P include H₆P₂Mo₁₈O₆₂, H₆P₂W₁₃Mo₅O₆₂, H₆P₂W₁₄Mo₄O₆₂, H₆P₂W₁₈O₆₂ and H₆P₂W₁₇MoO₆₂. It will be understood that phosphorus can be substituted for one or more other non-metal hetero atoms discussed herein. Reference to Keggin and Dawson anions herein may also include lacunary anions (i.e. those having a fragment of the structure missing). Exemplary Keggin lacunary anions are XM₁₁O₃₉ⁿ⁻ and XM₉O₃₄ⁿ⁻.

In an embodiment, the heteropoly acid has a Keggin structure.

The heteropoly acid is phosphotungstic acid. Other heteropoly acids include silicotungstic acid, phosphomolybdic acid, silicomolybdic acid, arsenotungstic acid, arsenomolybdic acid, borotungstic acid, boromolybdic acid, germanotungstic acid, germanomolybic acid, phosphotungsticmolybdic acid, silicotungsticmolybdic acid, arsenotungsticmolybdic acid, borotungsticmolybdic acid, and germanotungsticmolybdic acid.

In a particularly suitable embodiment, the heteropoly acid is a mixture of phosphotungstic acid, phosphomolybdic acid and phosphotungsticmolybdic acid. Suitably, the heteropoly acid is phosphotungstic acid. More suitably, the phosphotungstic acid has the Keggin structure.

It will be understood that the heteropoly acid may be part of a catalytic complex.

Also described herein is a heteropoly acid obtainable, obtained or directly obtained by a process for preparing a heteropoly acid defined herein.

The heteropoly acid may be provided on a support. Any suitable support material may be used. Exemplary supports include alumina (e.g. α-Al₂O₃ or γ-Al₂O₃), silica, alumina-silica, titania, zeolite, kaolinite, clay, active carbon, silicon carbide, and mixtures thereof. Suitably, the heteropoly acid is provided on an alumina support.

When the heteropoly acid is supported on a suitable support, the support constitutes 0.1-90% by weight of the total weight of the supported heteropoly acid. Suitably, the support constitutes 0.1-30% by weight of the total weight of the supported heteropoly acid. More suitably, the support constitutes 15-25% by weight of the total weight of the supported heteropoly acid.

In another embodiment, step a) comprises contacting the unsaturated organic compound with a mixed catalyst, wherein the mixed catalyst comprises one or more heteropoly acids and/or support. For example, step a) may comprise contacting the unsaturated organic compound with a mixture of H₃PW₁₂O₄₀ and H₃PMo₁₂O₄₀. Alternatively, step a) may comprise contacting the unsaturated organic compound with a mixture of H₃PW₁₂O₄₀ and TiO₂.

The volume ratio of heteropoly acid to unsaturated organic compound in step a) may be from 10:1 to 1:100. Suitably, the volume ratio of heteropoly acid to unsaturated organic compound is from 1:1 to 1:15.

In an embodiment, the heteropoly acid is phosphotungstic acid and has a BET surface area of ≥3 m²/g. More suitably, the heteropoly acid is phosphotungstic acid and has a BET surface area of ≥5 m²/g. Even more suitably, the heteropoly acid is phosphotungstic acid and has a BET surface area of 5-10 m²/g. The BET surface area may be calculated by N₂-physisorption analysis.

In an embodiment, the heteropoly acid is phosphotungstic acid and has a pore volume of 0.1-1.0 ml/g. More suitably, the heteropoly acid is phosphotungstic acid and has a pore volume of 0.2-0.8 ml/g. Even more suitably, the heteropoly acid is phosphotungstic acid and has a pore volume of 0.2-0.6 ml/g. Pore volume may be calculated using any suitable technique known in the art, such as, for example, techniques described in Fuel Processing Technology 135, 2015, 195-202.

In an embodiment, the heteropoly acid is phosphotungstic acid and has a density of 0.1-20 g/ml. More suitably, the heteropoly acid is phosphotungstic acid and has a density of 0.5-15 g/ml. Even more suitably, the heteropoly acid is phosphotungstic acid and has a density of 1-10 g/ml. Density may be calculated using any suitable technique known in the art, such as, for example, techniques described in Stage 6 Harmonization Text Notice publication on "Bulk Density and Tapped Density" found on the U.S. Pharmacopeial website (http://www.usp.org/usp-nf/harmonization/stage-6).

In another embodiment, the heterpoly acid is phosphotungstic acid and absorbs UV-vis light having a wavelength of between 250 and 450 nm.

The C=C bond migration process of the invention requires light of a wavelength of less than or equal to 700 nm. This encompasses visible light (approximately 400 nm to approximately 700 nm) and ultra violet light (approximately 10 nm to approximately 400 nm). In an embodiment, step a) is conducted in the presence of electromagnetic radiation having a wavelength of 10-700 nm. Suitably, step a) is conducted in the presence of electromagnetic radiation having a wavelength of 200-700 nm. More suitably, step a) is conducted in the presence of electromagnetic radiation having a wavelength of 300-700 nm. Even more suitably, step a) is conducted in the presence of electromagnetic radiation having a wavelength of 350-600 nm. Yet more suitably, step a) is conducted in the presence of electromagnetic radiation having a wavelength of 250-600 nm. Most suitably, step a) is conducted in the presence of electromagnetic radiation having a wavelength of 260-380 nm.

In an embodiment, step a) is conducted in the presence of electromagnetic radiation having a wavelength of less than 600 nm, suitably, less than 400 nm and, most suitably, less than 350 nm.

In another embodiment, the unsaturated organic compound and heteropoly acid of step a) of the present process are irradiated with electromagnetic radiation having a wavelength of less than or equal to 700 nm. Suitably, the unsaturated organic compound and heteropoly acid of step a) of the present process are irradiated with electromagnetic radiation provided from an irradiation (light) source, such as, for example, a lamp (e.g. a Xenon lamp).

It will be appreciated by the person skilled in the art that the term "irradiated" used herein refers to the exposure of the unsaturated organic compound and heteropoly acid of step a) of the present process to electromagnetic radiation which is greater in intensity than the electromagnetic radiation present under standard ambient conditions (i.e. the electromagnetic radiation provided by sunlight and room lighting). Accordingly, it will be readily understood that the unsaturated organic compound and heteropoly acid of step a) of the present process are, in the context of the present invention, only irradiated with electromagnetic radiation when an irradiation (light) source, such as, for example, a lamp (e.g. a Xenon lamp), is placed in close proximity to, or in directed contact with, a reaction vessel comprising the unsaturated organic compound and heteropoly acid of step a).

The irradiation (light) source used to irradiate the unsaturated organic compound and the heteropoly acid of step a) of the present process may have any suitable power output. Suitably, the irradiation (light) source used to irradiate the unsaturated organic compound and the heteropoly acid of step a) of the present process has a power output of between 200 Watts and 1000 Watts. More suitably, the irradiation (light) source used to irradiate the unsaturated organic compound and the heteropoly acid of step a) of the present process has a power output of between 200 Watts and 600 Watts. Yet more suitably, the irradiation (light) source used to irradiate the unsaturated organic compound and the heteropoly acid of step a) of the present process has a power output of between 200 Watts and 400 Watts. Most suitably, the irradiation (light) source used to irradiate the unsaturated organic compound and the heteropoly acid of step a) of the present process has a power output of 300 Watts.

In an embodiment, the irradiation (light) source is located within 50 cm of the reaction vessel comprising the unsaturated organic compound and the heteropoly acid of step a) of the present process. Suitably, the irradiation (light) source is located within 20 cm of the reaction vessel comprising the unsaturated organic compound and the heteropoly acid of step a) of the present process. More suitably, irradiation (light) source is located within 10 cm of the reaction vessel comprising the unsaturated organic compound and the heteropoly acid of step a) of the present process. Yet more suitably, the irradiation (light) source is located within 5 cm of the reaction vessel comprising the unsaturated organic compound and the heteropoly acid of step a) of the present process.

In another embodiment, the irradiation (light) source is in direct contact with the reaction vessel comprising the unsaturated organic compound and the heteropoly acid of step a) of the present process.

In yet another embodiment, the irradiation (light) source is located within the reaction vessel comprising the unsaturated organic compound and the heteropoly acid of step a) of the present process.

In a particular embodiment, the irradiation (light) source is a 300 W Xenon lamp.

To allow the irradiation (light) source to irradiate the unsaturated organic compound and the heteropoly acid of step a) of the present process it will be understood that the reaction vessel will allow electromagnetic radiation with a wavelength of between 10 nm to 700 nm to pass through it. Accordingly, the reaction vessel will not significantly absorb electromagnetic radiation with a wavelength of between 200 nm to 700 nm. Suitably, the reaction vessels used in step a) of the present process is a quartz reaction vessel.

Step a) may be conducted with or without a solvent. In an embodiment, the unsaturated organic compound may be provided neat (i.e. without a separate solvent). A non-limiting example of an unsaturated organic compound that can be used neat in the process of the invention is 1-hexene. Alternatively, the unsaturated organic compound may be provided as a solution in any suitable solvent.

In contrast to prior art techniques requiring elevated temperatures, step a) of the present process may be conducted at any temperature below the boiling point of the unsaturated organic compound (and any additional solvent, where present). In an embodiment, step a) of the present process may be conducted at a temperature ranging from 18°C-110°C.

Step a) of the present process may be carried out at any suitable pressure (i.e. at both atmospheric and elevated pressure). Suitably, and contrary to prior art techniques requiring elevated pressures, step a) of the present process may be carried out at atmospheric pressure. That is to say, step a) of the present process may be conducted at a pressure of between 100 kPa and 1000 kPa, suitably at a pressure of between 100 kPa and 500 kPa, and most suitably at a pressure of between 100 kPa and 150 kPa.

When compared with prior art techniques, the present process allows the reaction time to be markedly reduced. In an embodiment, step a) may be performed over a period of 30 to 720 minutes. Suitably, step a) may be performed over a period of 60 to 600 minutes. More suitably, step a) may be performed over a period of 60 to 420 minutes. Even more suitably, step a) may be performed over a period of 60 to 240 minutes. Even more suitably, step a) may be performed over a period of 120 to 240 minutes. Although the present process allows some C=C double bond migration to be achieved in a comparatively reduced period of time, C=C double bond migration may nonetheless occur to a greater extent when longer reaction periods are used.

In certain embodiments, step a) may be performed over a period of 1 to 450 minutes. Suitably, step a) may be performed over a period of 5 to 450 minutes. More suitably, step a) may be performed over a period of 10 to 450 minutes. Even more suitably, step a) may be performed over a period of 10 to 100 minutes. Even more suitably, step a) may be performed over a period of 10 to 60 minutes.

### Preparation of heteropoly acids

Also described herein is a process for the preparation of a heteropoly acid, the process comprising the steps of:
a) admixing:
   i. water,
   ii. a metal source comprising either or both of tungsten and molybdenum,
   iii. a non-metal source comprising one or more of silicon, phosphorus, arsenic and germanium, and
   iv. at least one water-soluble polar organic compound;
b) adjusting the pH of the mixture of step a) to ≤2;
c) isolating the product formed in step b);
d) drying the isolated product; and
e) optionally calcining the dried isolated product.

When compared with prior art techniques, the process allows for the preparation of heteropoly acids having considerably higher surface area. The improved surface area increases the efficiency of the heteropoly acid when it is used in catalytic processes.

The water-soluble polar organic compound may be selected from an organic acid, an alcohol, a glycol, glycine, an amine, or a mixture thereof. Suitably, the water-soluble polar organic compound is selected from citric acid, acetic acid, oxalic acid, urea, or a mixture thereof. The water-soluble polar organic compound may be citric acid. The quantity of water-soluble polar organic compound is between 5 and 50 wt.% relative to the total weight of the mixture of step a), neglecting the mass of water.

Step b) may comprise adjusting the pH of the mixture of step a) to ≤2. Suitably, step b) comprises adjusting the pH of the mixture of step a) to ≤1. More suitably, step b) comprises adjusting the pH of the mixture of step a) to 0-0.5. Reducing the pH to 2 or below causes the formation a heteropoly acid having the Keggin structure, which is preferred. The pH may be reduced in step b) with any suitable acid. Most suitably, the acid is HCI.

It will be understood that the metal source may be any molecule comprising tungsten or molybdenum. The tungsten or molybdenum may be present in an uncharged state, or in any suitable charged state. Similarly, the non-metal source may be any molecule comprising silicon, phosphorus, arsenic or germanium, present in either an uncharged state or in any suitable charged state.

The metal source may be a source of a metal in its 6+ oxidation state.

The non-metal source may comprise phosphorus. Suitably, the non-metal source is phosphoric acid.

The metal source may be an alkali metal tungstate or an alkali metal molybdate, or a mixture thereof. Suitably the metal source is sodium tungstate or sodium molybdate, or a mixture thereof. The tungstate or molybdate may be present in hydrated form (e.g. Na₂WO₄•2H₂O; or Na₂MoO₄•2H₂O).

Step a) may be conducted at a temperature of 30-90°C.

In another embodiment, after step b) but prior to step c), the mixture resulting from step b) is aged. Aging may involve subjecting the mixture resulting from step b) to high temperatures and/or pressures. Suitably, the mixture resulting from step b) is hydrothermally aged at a temperature of ≥ 90°C. More suitably, the mixture resulting from step b) is hydrothermally aged at a temperature of ≥ 100°C. When utilised, an aging step can improve the crystallinity of the resulting heteropoly acid, as well as increasing the dispersion of these crystallites.

After step b) (and any aging step), but prior to step c), the mixture may be treated with an oxidizing agent. Suitably, the oxidizing agent is hydrogen peroxide or bromine.

The heteropoly acid can be isolated in step c) via any suitable technique (e.g. filtration or rotary evaporation of solvent). It will be understood that the isolation step may comprise one or more separation or extraction steps. Step c) may comprise extracting the product of step b) into a suitable solvent, followed by removal of the solvent. Suitably, the product of step b) is extracted with diethyl ether. The product of step b) may be isolated by recrystallization.

The isolated product may be dried at any suitable temperature for any suitable duration. Step d) may comprise drying the isolated product at a temperature of ≥ 90°C. More suitably, step d) comprises drying the isolated product at a temperature of ≥ 120°C. Even more suitably, step d) comprises drying the isolated product at a temperature of ≥ 200°C. Step d) may comprise drying the isolated product at a temperature of ≥ 300°C

In addition to the drying step, the dried isolated product may be calcined. Calcination may be performed in static air at a temperature of up to 800°C for more than 1 hour. Where the drying step d) occurs at a temperature that is particularly high (e.g. ≥ 300°C) a calcining step may be omitted.

The heteropoly acid may be provided on a suitable support material. This may be achieved by mixing the heteropoly acid prepared via the present process with a suitable support material with or without a solvent. Alternatively, a supported heteropoly acid may be prepared by including a suitable support material in step a) of the present process, thereby forming the supported heteropoly acid *in-situ.* Any suitable support material may be used, including those recited herein. Suitably the support material is Al₂O₃.

Also described herein is a heteropoly acid obtainable, obtained or directly obtained by a process defined herein.

The heteropoly acids obtainable by the process described herein may present numerous advantages over commercially available heteropoly acids. Perhaps most notably, these heteropoly acids have considerably higher surface areas than commercially available heteropoly acids. The increased surface area renders the heteropoly acid more efficient when used as a catalyst.

The heteropoly acid obtainable by the process described herein may have a BET surface area of ≥3 m²/g. More suitably, the heteropoly acid has a BET surface area of ≥5 m²/g. Even more suitably, the heteropoly acid has a BET surface area of 5-10 m²/g. The BET surface area may be calculated by N₂-physisorption analysis.

The heteropoly acid obtainable by the process described herein may have a pore volume of 0.1-1.0 ml/g. More suitably, the heteropoly acid has a pore volume of 0.2-0.8 ml/g. Even more suitably, the heteropoly acid has a pore volume of 0.2-0.6 ml/g. Pore volume may be calculated using any suitable technique known in the art, such as, for example, techniques described in Fuel Processing Technology 135, 2015, 195-202.

The heteropoly acid obtainable by the process described herein may have a density of 0.1-20 g/ml. More suitably, the heteropoly acid has a density of 0.5-15 g/ml. Even more suitably, the heteropoly acid has a density of 1-10 g/ml. Density may be calculated using any suitable technique known in the art, such as, for example, techniques described in Stage 6 Harmonization Text Notice publication on "Bulk Density and Tapped Density" found on the U.S. Pharmacopeial website (http://www.usp.org/usp-nf/harmonization/stage-6).

The heterpoly acid obtainable by the process described herein may absorb UV-vis light having a wavelength of between 250 and 450 nm.

### EXAMPLES

Non-limiting examples of the invention will now be described, for the purpose of illustration only, with reference to the accompanying figures, in which:
Fig. 1 shows the Keggin (left) and Dawson (right) structures of heteropoly acids.
Fig. 2 is a flow diagram illustrating the synthesis of the heteropoly acid prepared in Example 1.
Fig. 3 shows the UV-vis absorption spectrum for the heteropoly acid prepared in Example 1.
Fig. 4 compares the XRD patterns of the heteropoly acid prepared in Example 1 (the "catalyst") with a commercially-available tungstophosphoric acid.
Figure 5 shows a surface electron microscope (SEM) image of the heteropoly acid prepared in Example 1 (the "catalyst").
Figure 6 shows a transmission electron microscope (TEM) image of the heteropoly acid prepared in Example 1 (the "catalyst").
Fig. 7 shows the gas chromatography-mass spectrometry (GC-MS) spectra of samples after double bond isomerization of oleic acid (OA) at different time points. A schematic illustration of the isomerization of oleic acid to vaccenic acid is also shown.

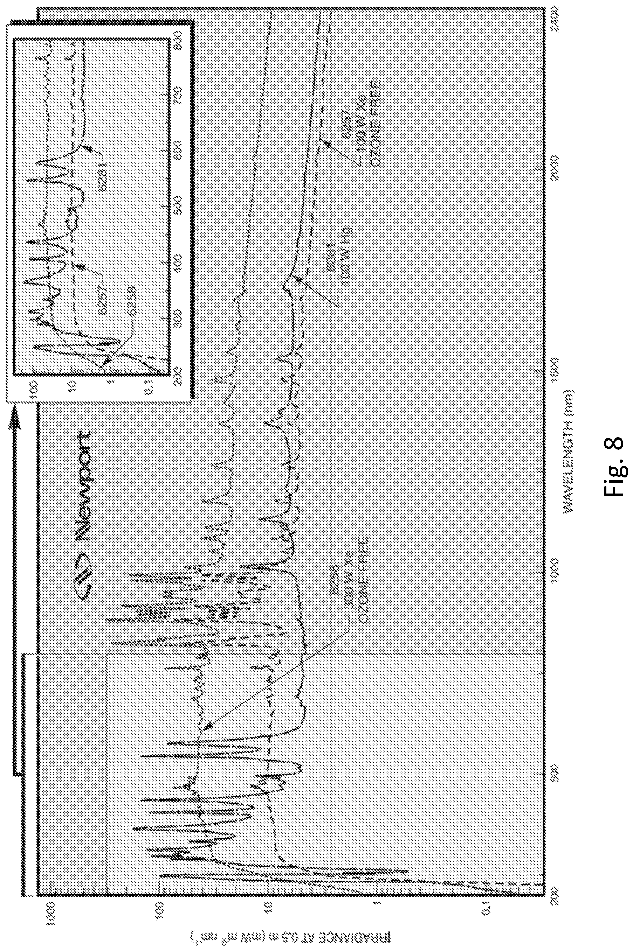

Fig. 8 shows the spectral irradiance of a Newport® 6258 lamp.
Fig. 9 shows the GC-MS spectrum at a retention time of between 10 and 15 minutes for the C=C isomerization of allylbenzene.
Fig. 10 shows the GC-MS spectra at a retention time of between 10.50 and 10.57 minutes for the C=C isomerization of allylbenzene at various time points. The spectra shown are an amplification of the first peak of the GC-MS spectrum displayed in Fig. 7).
Fig. 11 shows the GC-MS spectra at a retention time of between 12.0 and 12.5 minutes for the C=C isomerization of allylbenzene. The spectra shown are an amplification of the second peak of the GC-MS spectrum displayed in Fig. 7).
Fig. 12 shows the GC-MS spectra at a retention time of between 13.5 and 14.2 minutes for the C=C isomerization of allylbenzene. The spectra shown are an amplification of the third peak of the GC-MS spectrum displayed in Fig. 7).
Figure 13 shows the IR spectra for samples before and sample after C=C isomerization of allylbenzene.

### Chemicals and Materials

2-propenylbenzene (allylbenzene), 98%, CAS 300-57-2, C₉H₁₀, MW: 118.18g•mol⁻¹ was purchased from Sigma-Aldrich Company Ltd. (The Old Brickyard New road, Gillingham Dorset SP8 4XT United Kingdom).

Phosphotungstic acid hydrate (PTA), self-prepared (see above), formula: H₃O₄₀PW₁₂•xH₂O

### Apparatus

### Universal ARC Lamp Housings

New port Oriel Product Line, 150 Long Beach Boulevard Stratford, CT 06615-0872.

| | |
|---|---|
| **Lamp:** | Xe |
| **Watt:** | 300W |
| **Ambient Temp.** | 40-60°C |
| **Lamp running time:** | 0min, 30min,60min,90min,120min,150min,180min,210min,240min, 270min, 300min. |

### Arc Lamp Supply:

- Model 69911;
- Arc Lamp: 6258;
- Lamp Description: OF 300W Xe;
- Power Range Watts: 240-330;
- Typical Voltage VDC: 20;
- Typical Current ADC: 15;
- Average Life (hours): 900;
- Lamp Housing: 50-500W (Research Housing);
- Lamp Socket Adapter: 66160.

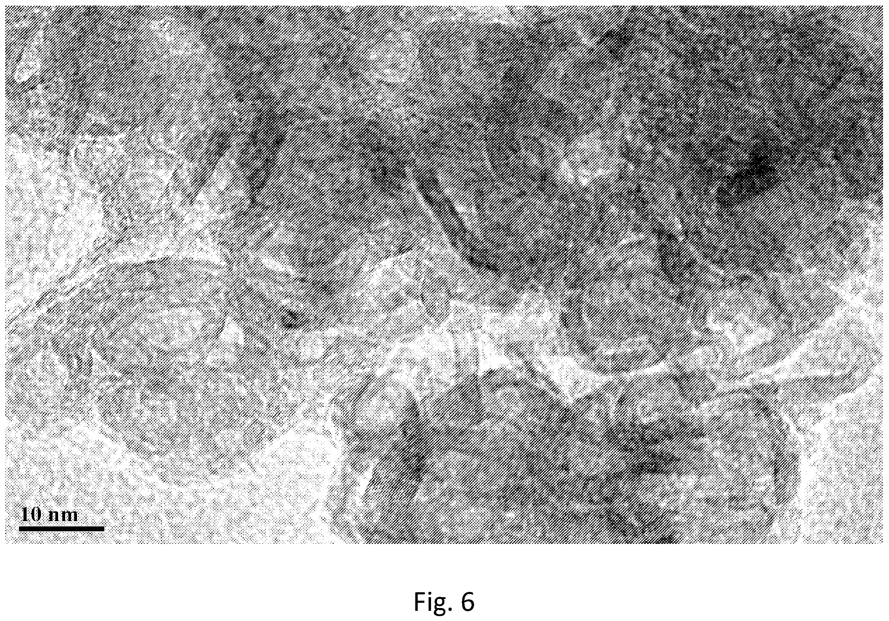

Figure 6 shows the spectral irradiance of a Newton® 6258 lamp (sourced from the Oriel Product Training, Spectral Irradiance Manual, Figure 6, page 24).

### GC-MS (GCMS-QP2010 SE, SHIMADZU)

The GC-MS used included a gas chromatograph (GC-2010plus, SHIMADZU), an autosampler (AOC-201, SHIMADZU) and a column (SHIM-5MS, Thickness: 0.25µm, Length: 30.0m, Diameter: 0.25mm).

The following conditions were used:
- Column Oven Temp.: 50°C;
- Injection Temp.: 200°C;
- Injection mode: split;
- Carrier Gas: He;
- Prim. Press: 500-900;
- Flow Control Mode: Linear Velocity;
- Pressure: 7.9kPa;
- Total Flow: 6.9 ml/min;
- Column Flow: 0.50ml/min;
- Linear Velocity: 25.6cm/sec;
- Purge Flow: 1.4 ml/min;
- Inject. Heat Port: INJT;
- Split Ratio: 100.0;
- Injection volume: 0.1µL.
The following temperature program of the GC-MS was used:

| | **Rate** | **Final Temp.** | **Hold Time(min)** |
|---|---|---|---|
| 0 | - | 50.0 | 5 |
| 1 | 5.00 | 100.0 | 1.00 |
| 2 | 20.00 | 250.00 | 30.00 |
| 3 | 0.00 | 0.00 | 0.00 |

### Example 1 - Preparation of heteropoly acid (H₃PW₁₂O₄₀, Keggin structure)

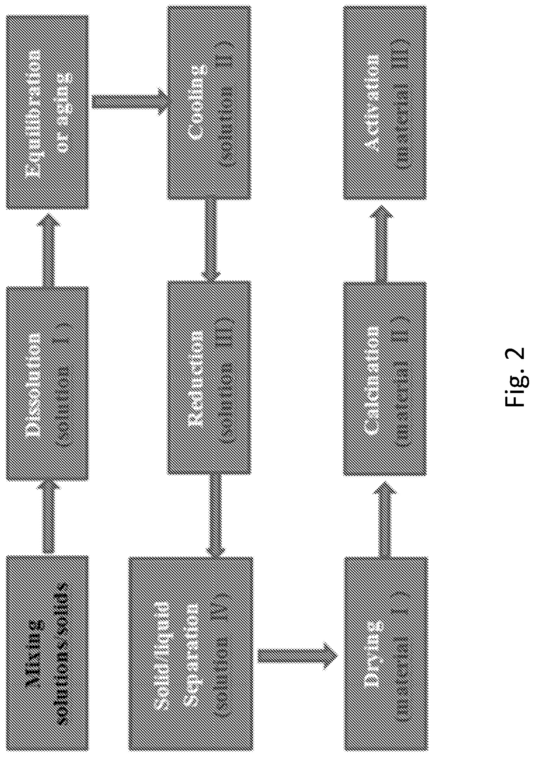

Referring to Fig. 2, "solution I" was obtained by mixing 50 g sodium tungstate (Na₂WO₄·2H₂O, Sigma-Aldrich), 5.6g concentrated phosphoric acid (H₃PO₄, 85%wt) and 46mL concentrated hydrochloric acid (HCl, 32%wt) and 4.2 g of citric acid under moderate magnetic stirring at temperature from 30 - 90°C for 3-20 minutes until it turned to shallow yellow. Next, "solution I" was left to naturally cooled to room temperature until it became colorless "solution II". If "solution II" became blue, hydrogen peroxide (H₂O₂, 3%wt, Sigma-Aldrich) or bromine (Br₂) was added to the solution until it became colorless ("solution III").

"solution III" and 40mL ethyl ether were added into a separating funnel, and then the funnel was then inverted repeatedly. The separating funnel is set aside to allow for the complete separation of the phases to form three layers. The bottom layer "solution IV" -heteropoly acid and ether compounds- is first removed for further extraction, the middle layer-sodium chloride (NaCl), and hydro chloride (HCl) aqueous solution-has been removed secondly, finally the upper layer-ethyl ether-is poured out through the top into another container.

After the "solution IV" -heteropoly acid and ether compounds-was extracted for 3-5 times, then dried in the air to form "material I", and calcined at 90-120 °C to become " material II" , then finally activated (e.g. calcined) to form "material III". Material II may, however, also be used in the catalytic process without any activation step.

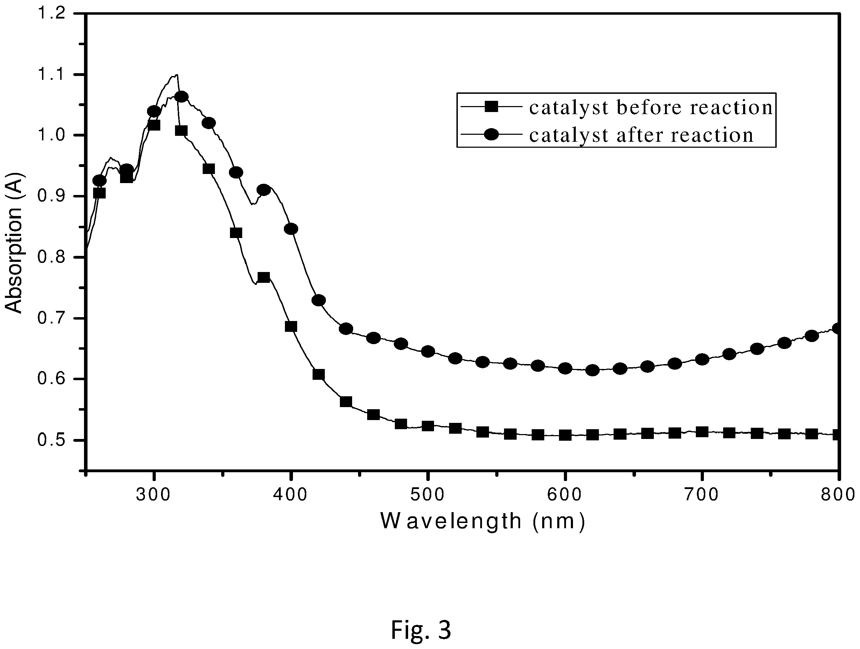

Fig. 3 shows the UV-vis absorption spectrum of the resulting phosphotungstic acid. Clearly the prepared catalyst has two UV-vis absorption peaks in the range extending to 400 nm, which can make use of blue and violet light directly.

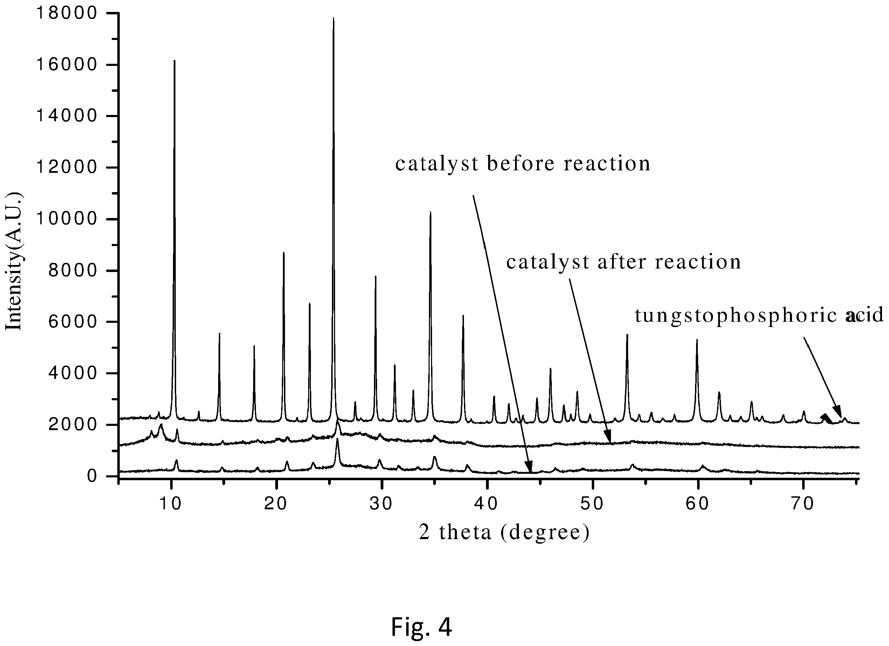

Fig. 4 compares the X-ray diffraction pattern of the phosphotungstic acid catalyst of Example 1 (the "catalyst") with that of a commercially available tungstophosphoric acid obtained from Tianjin Zhenhua, Tianjin, China heteropoly acid (H₃PW₁₂O₄₀, Keggin structure). The similarity in peaks is evident, although the weaker diffraction peaks of the prepared catalyst are perhaps indicative of smaller crystallite particles.

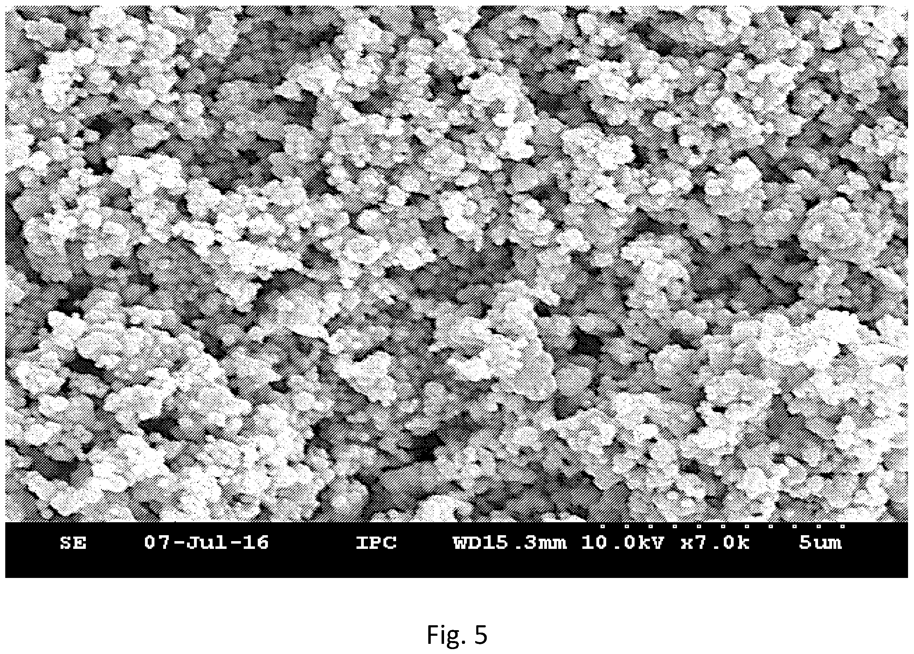

Fig. 5 shows an SEM image and Fig. 6 shows a TEM image of the of the heteropoly acid prepared in Example 1 (the "catalyst").

### Example 2 - migration of C=C double bonds

### Example (i) - Attempted isomerization of 1-hexene with photocatalysts under irradiation of UV-vis and visible 300W Xenon light, respectively.

2.0 grams of a tungsten-phosphoric acid catalyst (H₃PW₁₂O₄₀, prepared in Example 1) were added to a quartz test tube equipped with a magnetic stirrer, maintained at a temperature of 90° C. 30mL of 1-hexene (available from Sigma-Aldrich) were also added to the test tube equipped with a magnetic stirrer, maintained at a temperature of 90°C. The contents were mixed continuously, and then irradiated under UV-vis and visible 300W Xenon lamp, respectively. Each series of samples, withdrawn after 60, 120, 180, 240, 300, 360, 420 minutes, were analyzed via gas chromatography-mass spectrometry (GC-MS) and nuclear magnetic resonance (NMR). The analysis showed that all of the 1-hexene both after 540 minutes under irradiation of UV-vis and visible 300W Xenon lamp, respectively, were isomerized to 70 percent of 2-hexene and 3-hexene.

### Example (ii) - Attempted isomerization of 1-octene with photocatalysts under irradiation of UV-vis and visible 300W Xenon light, respectively.

2.0 grams of a tungsten phosphoric acid (H₃PW₁₂O₄₀, prepared in Example 1 catalyst and of 1.0 grams of molybdenum phosphoric acid catalyst (H₃PMoO₆₀, Keggin structure, prepared by East China University of Science and Technology) were added to a quartz test tube equipped with a magnetic stirrer, maintained at a temperature of 90° C. 15 mL of 1-octene (available from Sigma-Aldrich) were also added to the test tube equipped with a magnetic stirrer, maintained at a temperature of 90°C. The contents were mixed continuously and then irradiated under UV-vis and visible 300W Xenon lamp, respectively. Each series of samples, withdrawn after 60, 120, 180, 240, 300, 360, 420 minutes, were analyzed via gas chromatography-mass spectrometry (GC-MS) and nuclear magnetic resonance (NMR). The analysis showed that all of 1-octene both after 540 minutes under irradiation of UV-vis and visible 300W Xenon lamp, respectively, were isomerized to 70 percent of 2-octene and 3-octene.

### Comparative Example (i) - Attempted isomerization of 1-hexene without photocatalyst under irradiation of UV-vis or visible 300W Xenon light, respectively.

15 mL of 1-hexene (available from Sigma-Aldrich) were added to a quartz test tube equipped with a magnetic stirrer, maintained at a temperature of 90° C. The solution was then irradiated under UV-vis and visible 300W Xenon lamp, respectively. Each sample, withdrawn after 480 minutes, was analyzed via gas chromatography-mass spectrometry (GC-MS) and nuclear magnetic resonance (NMR). The analysis showed that the 1-hexene under both irradiation of UV-vis and visible 300W Xenon lamp, respectively had exhibited no isomerization to 2-hexene.

### Comparative Example (ii) - Attempted isomerization of 1-hexene with catalysts in the dark.

1.0 grams of a tungsten phosphoric acid catalyst (H₃PW₁₂O₄₀, prepared in Example 1) were added to a quartz test tube equipped with a magnetic stirrer, maintained at a temperature of 23° C. 15 mL of 1-hexene (available from Sigma-Aldrich) were also added to the test tube equipped with a magnetic stirrer, maintained at a temperature of 90°C. The contents were mixed continuously in the dark. One sample, withdrawn after 240 hours, was analyzed via gas chromatography-mass spectrometry (GC-MS) and nuclear magnetic resonance (NMR). The analysis showed that the 1-hexene with catalyst in the dark for 240 hours had exhibited no isomerization to 2-hexene.

### Comparative Example (iii) - Attempted isomerization of 1-octene without photocatalyst under irradiation of UV-vis or visible 300W Xenon light, respectively.

15 mL of 1-octene (available from Sigma-Aldrich) were added to a quartz test tube equipped with a magnetic stirrer, maintained at a temperature of 90° C, and was then irradiated under UV-vis and visible 300W Xenon lamp, respectively. Each sample, withdrawn after 480 minutes, was analyzed via gas chromatography-mass spectrometry (GC-MS) and nuclear magnetic resonance (NMR). The analysis showed that the 1-octene both under irradiation of UV-vis and visible 300W Xenon lamp, respectively had exhibited no isomerization to 2-octene or 3-octene.

### Comparative Example (iv) - Attempted isomerization of 1-octene with catalysts in the dark.

1.0 grams of a tungsten phosphoric acid (H₃PW₁₂O₄₀, prepared in Example 1 catalyst were added to a quartz test tube equipped with a magnetic stirrer, maintained at a temperature of 23° C. 15 mL of 1-octene (available from Sigma-Aldrich) were also added to the test tube equipped with a magnetic stirrer, maintained at a temperature of 90°C. The contents were mixed continuously in the dark. One sample, withdrawn after 240 hours, was analyzed via gas chromatography-mass spectrometry (GC-MS) and nuclear magnetic resonance (NMR). The analysis showed that the 1-octene with catalyst in the dark for 240 hours had exhibited no isomerization to 2-octene or 3-octene. Instead, all of the octene had been polymerized to di-or tri-polymer.

### Example (v) - Attempted isomerization of oleic acid with photocatalysts under irradiation of visible 300W Xenon light.

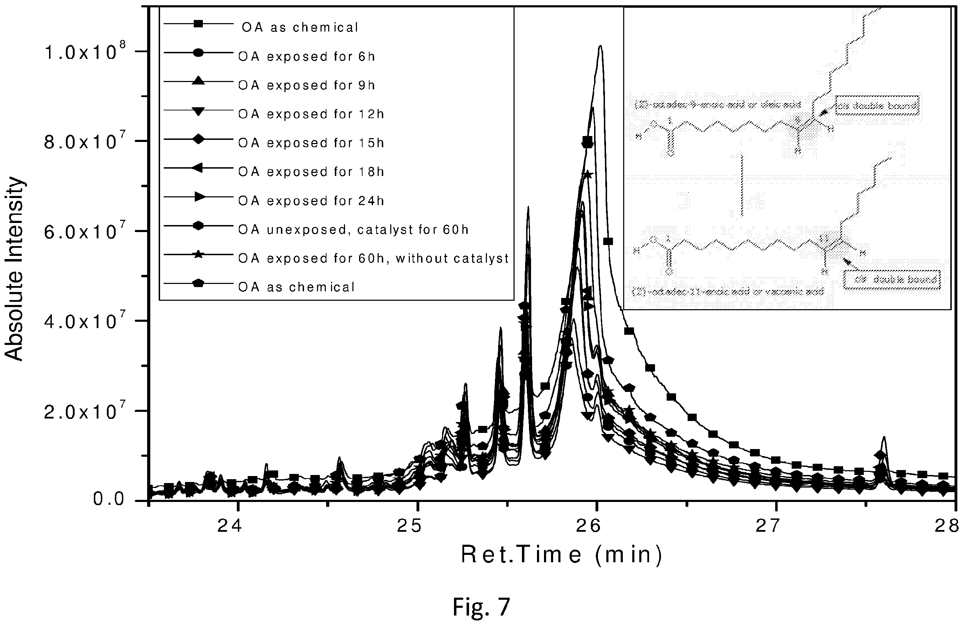

4.0 grams of a tungsten-phosphoric acid catalyst (H₃PW₁₂O₄₀, prepared in Example 1) were added to a quartz test tube equipped with a magnetic stirrer, maintained at a temperature of 80° C. 40mL of oleic acid (available from Sigma-Aldrich) were also added to the test tube equipped with a magnetic stirrer, maintained at a temperature of 80°C. The contents were mixed continuously, and then irradiated with a UV-visible 300W Xenon lamp. Each series of samples, withdrawn after 6, 9, 12, 15, 18 and 24 hours, were analyzed via gas chromatography-mass spectrometry (GC-MS). The analysis showed that oleic acid was isomerized to vaccenic acid (see Figure 7).

### Example 3 - Comparison of heteropoly acid catalyst performance

Table 1 below compares the catalytic efficiency of the heteropoly acid of Example 1 with a commercially available benchmark, when used in the isomerisation of CH₂=CH-(CH₂)₁₃-CH₃.

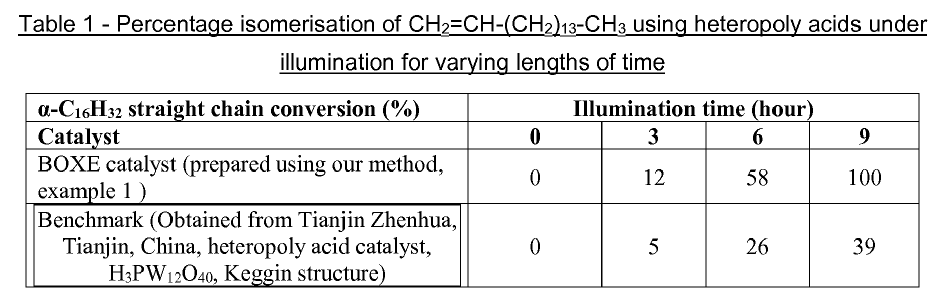

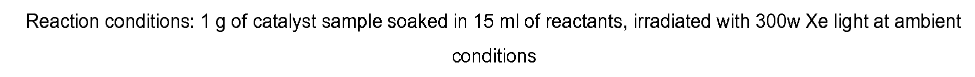

As is clear from the data presented in Table 1, the catalyst of Example 1 had isomerised more CH₂=CH-(CH₂)₁₃-CH₃ than the commercial benchmark at every time interval, and had effected complete conversion of all of the CH₂=CH-(CH₂)₁₃-CH₃ starting material after 9 hours.

Tables 2 to 8 below compare the catalytic efficiency of the heteropoly acid of Example 1 in the isomerisation of 1-hexene (1-C₆H₁₂), 1-heptene (1-C₇H₁₄), 1-octene (1-C₈H₁₆), 1-decene (1-C₁₀H₂₀), 1-dodecene (1-C₁₂H₂₄), 1-tetradecene (1-C₁₄H₂₈) and 1-octadecene (1-C₁₈H₃₆) respectively, when irradiated with visible light and UV-vis light and when conducted in the dark.

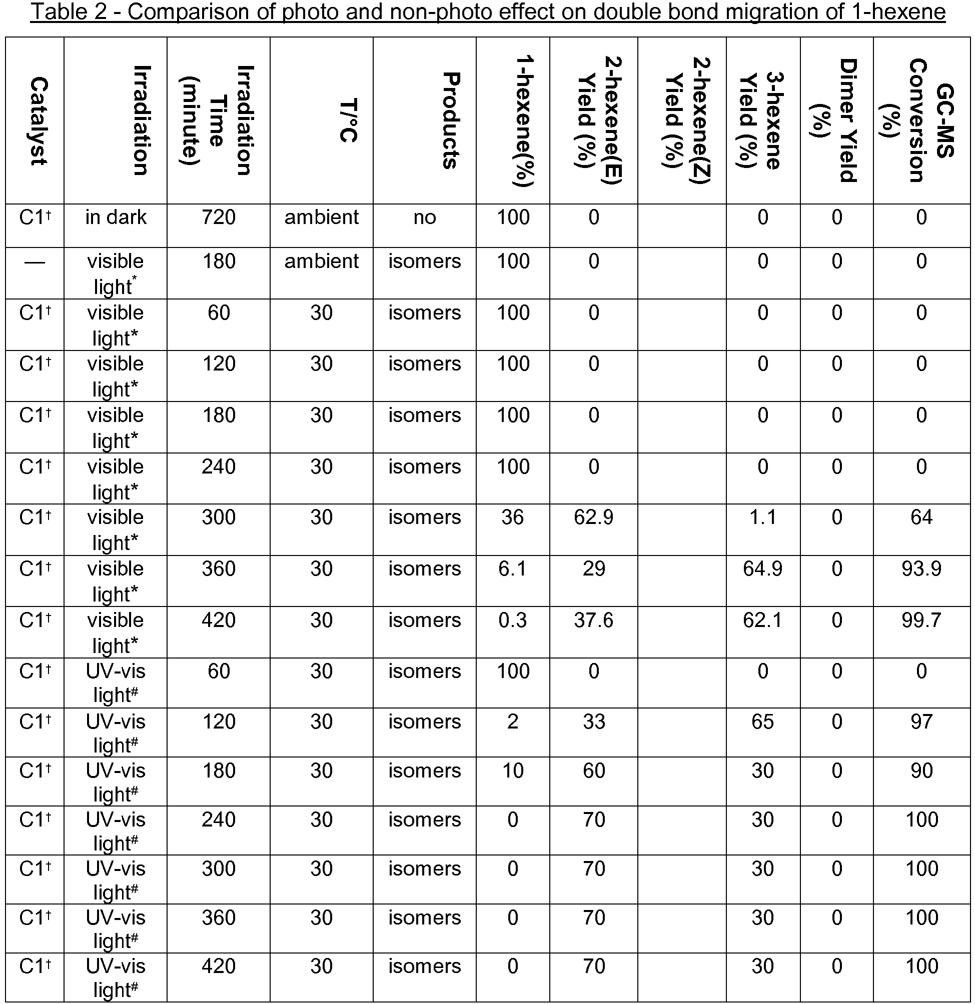

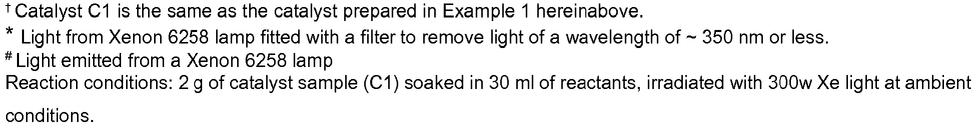

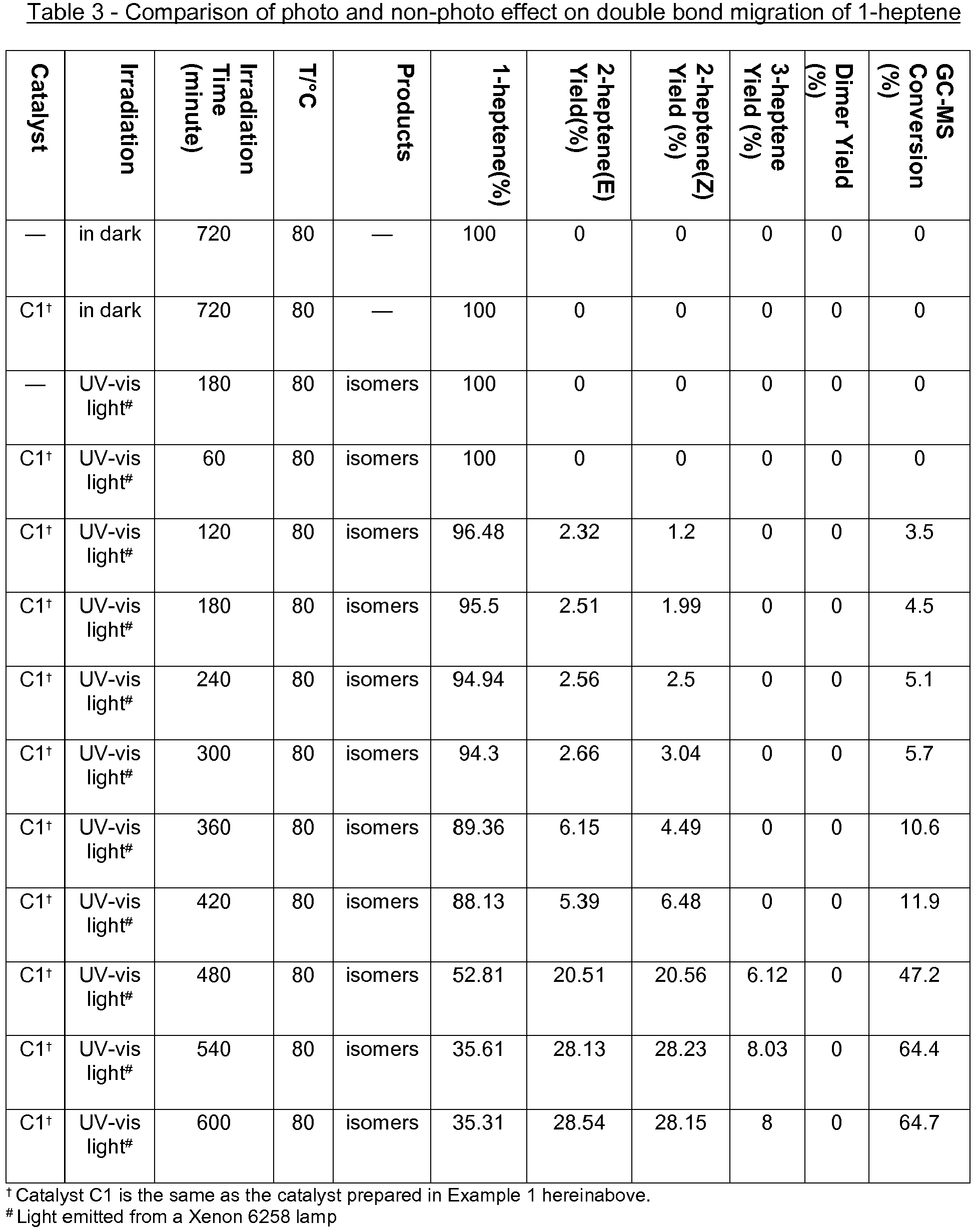

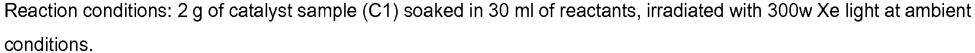

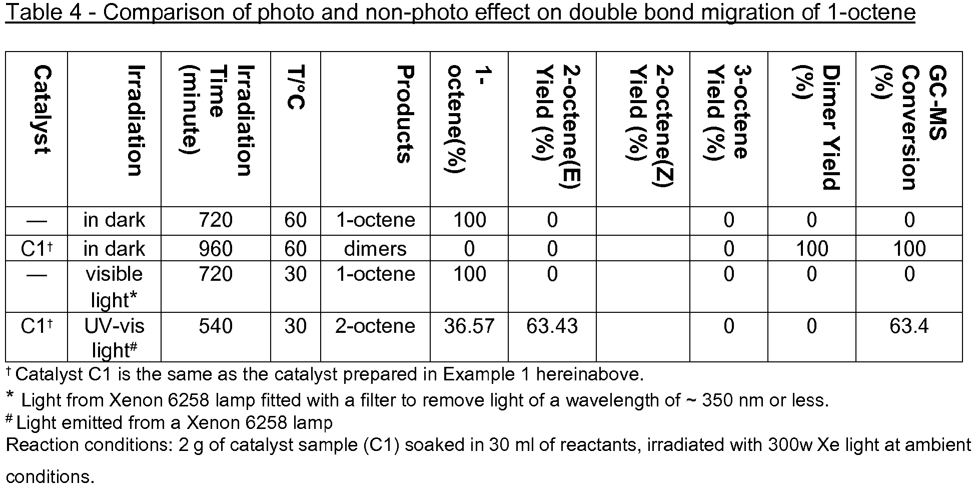

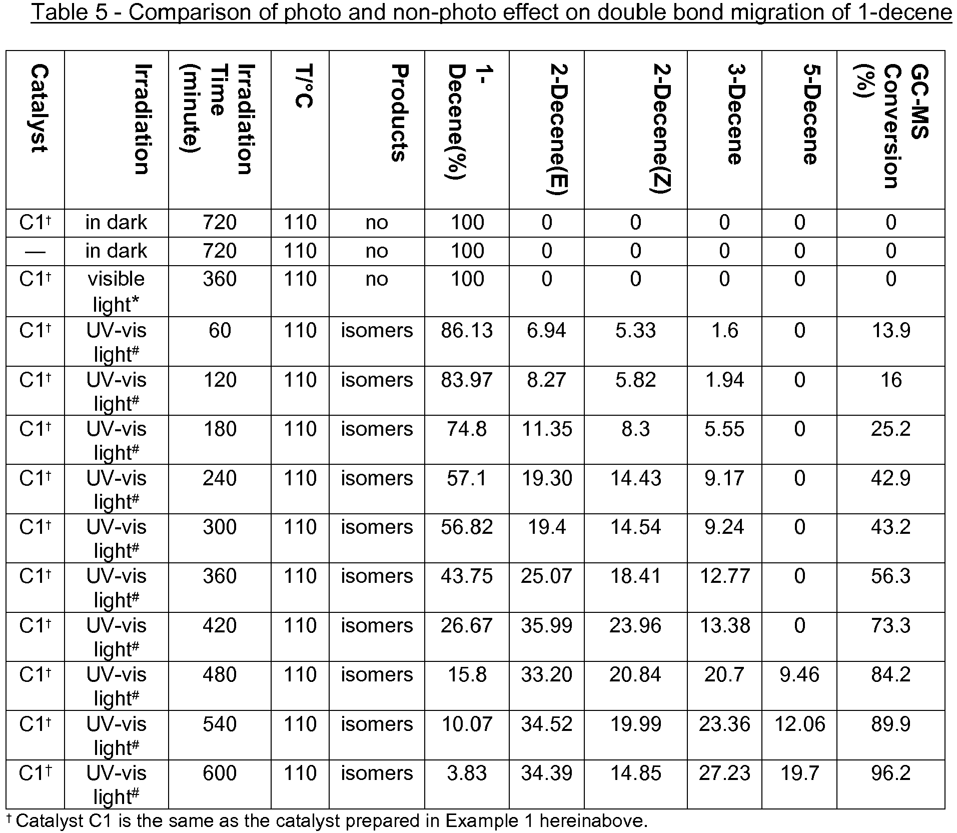

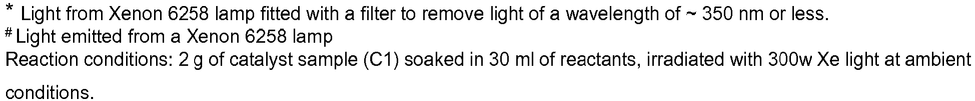

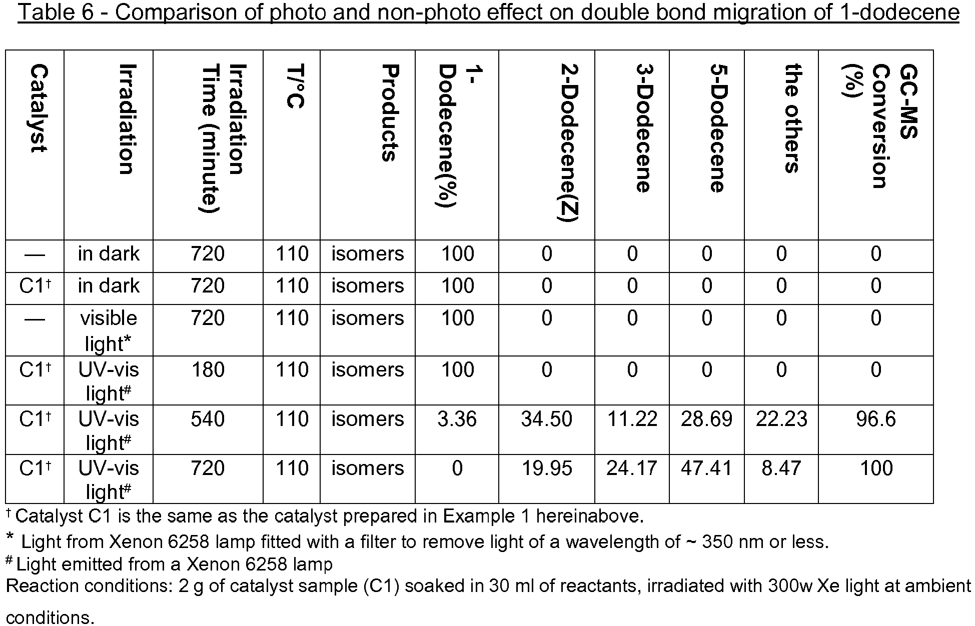

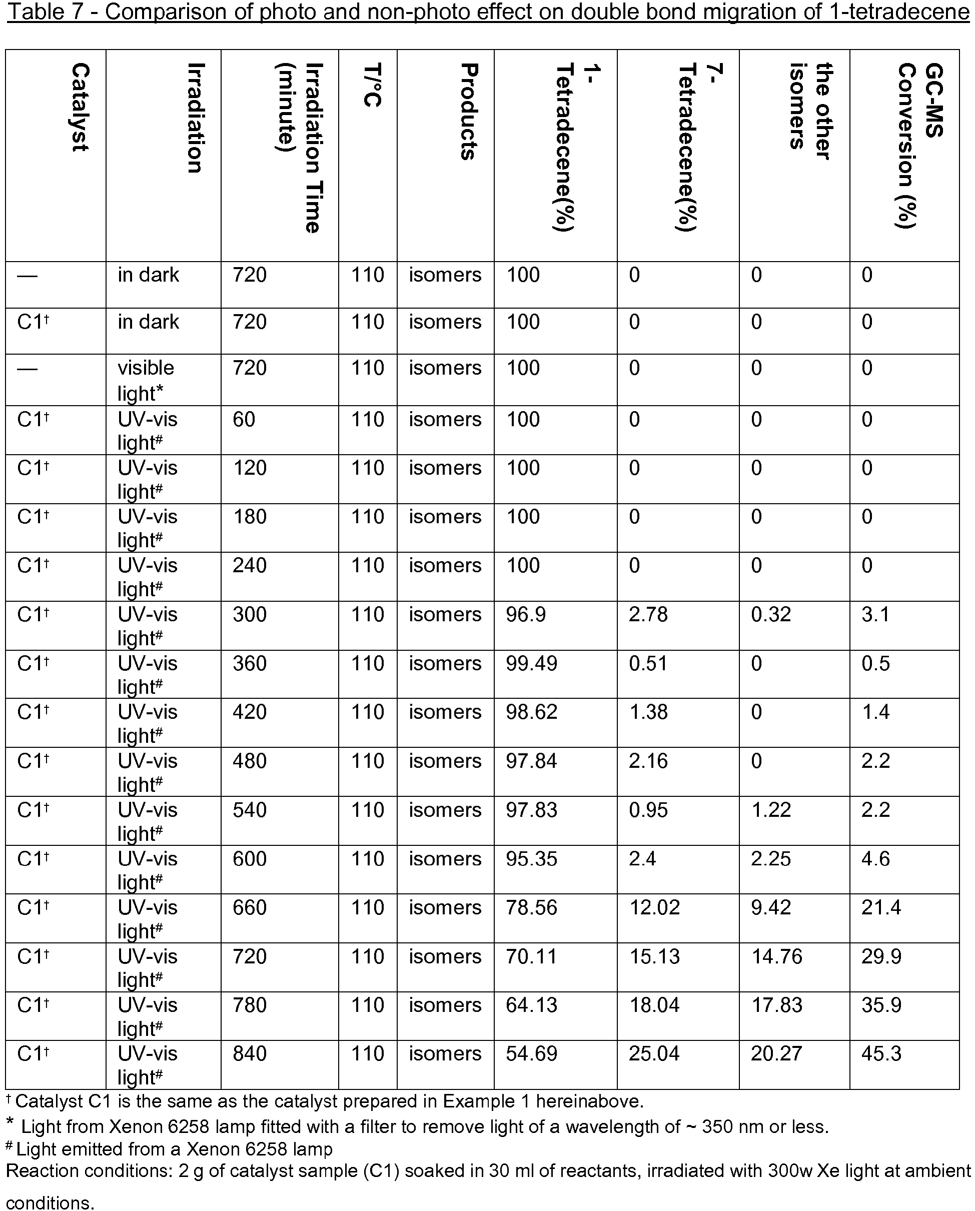

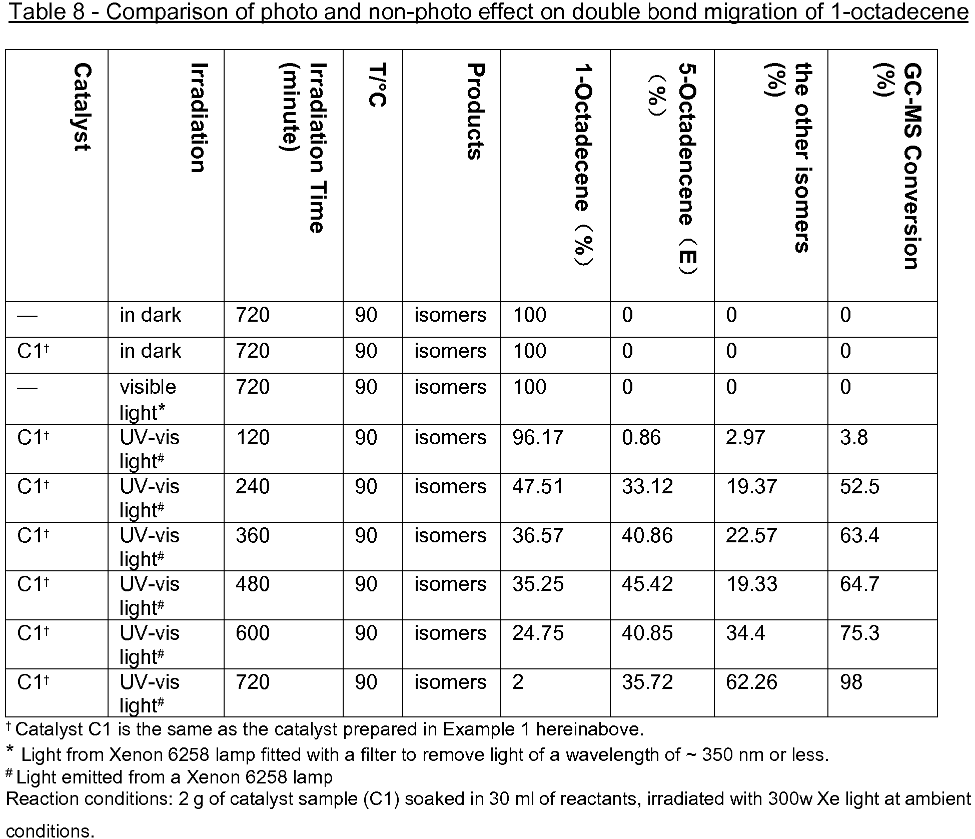

### Example 4 - Isomerization of allylbenzene

### Procedure

### Double bond isomerization reaction of allylbenzene

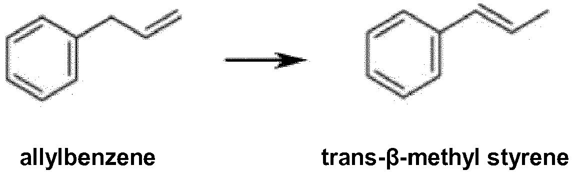

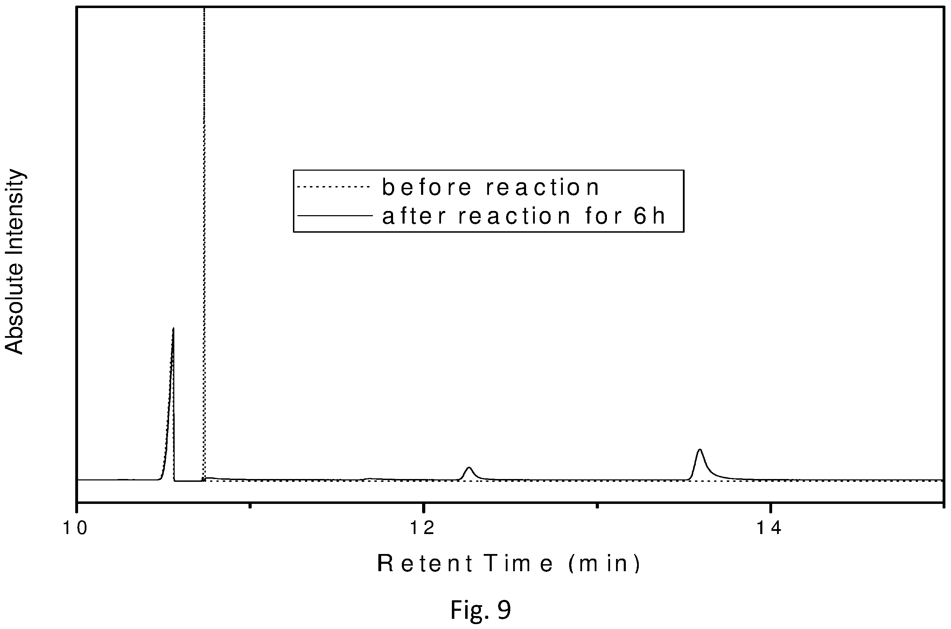

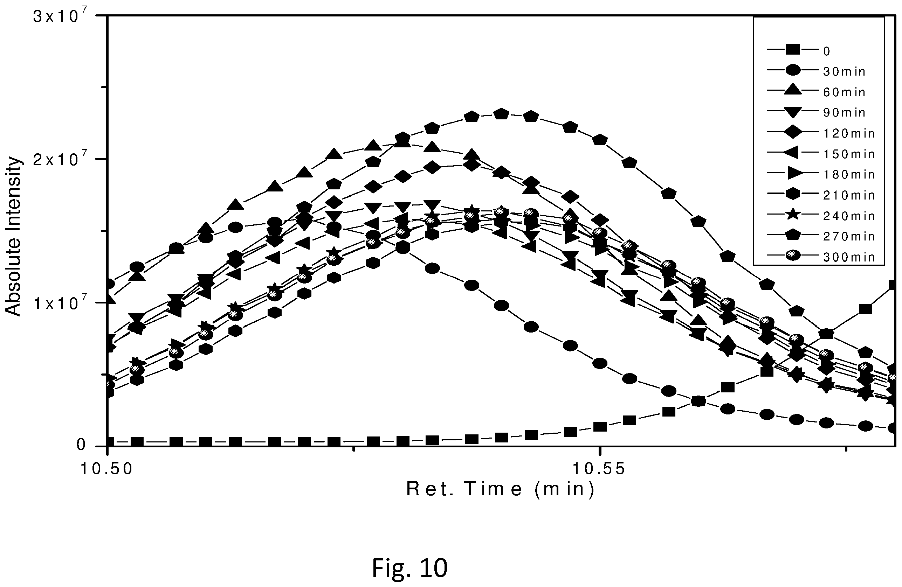

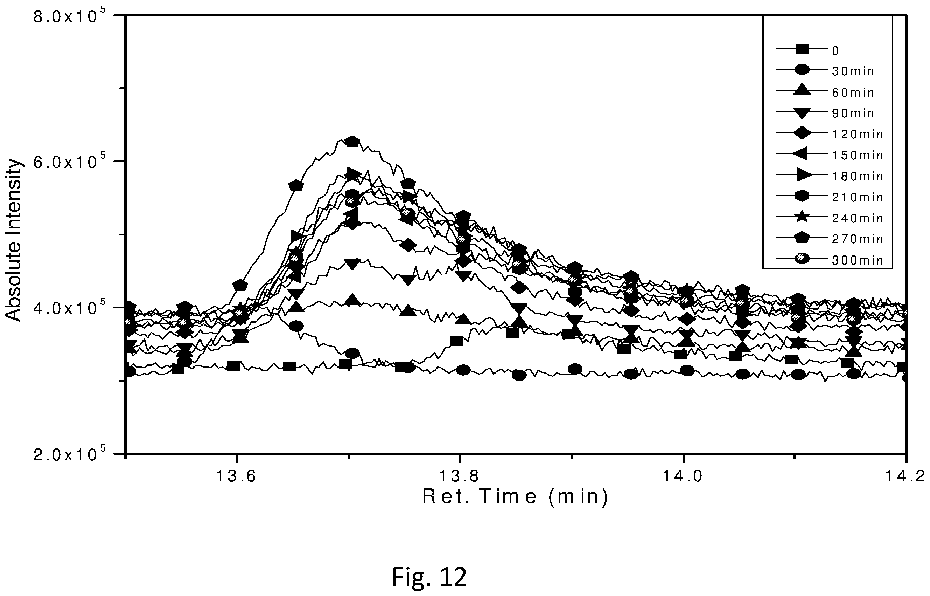

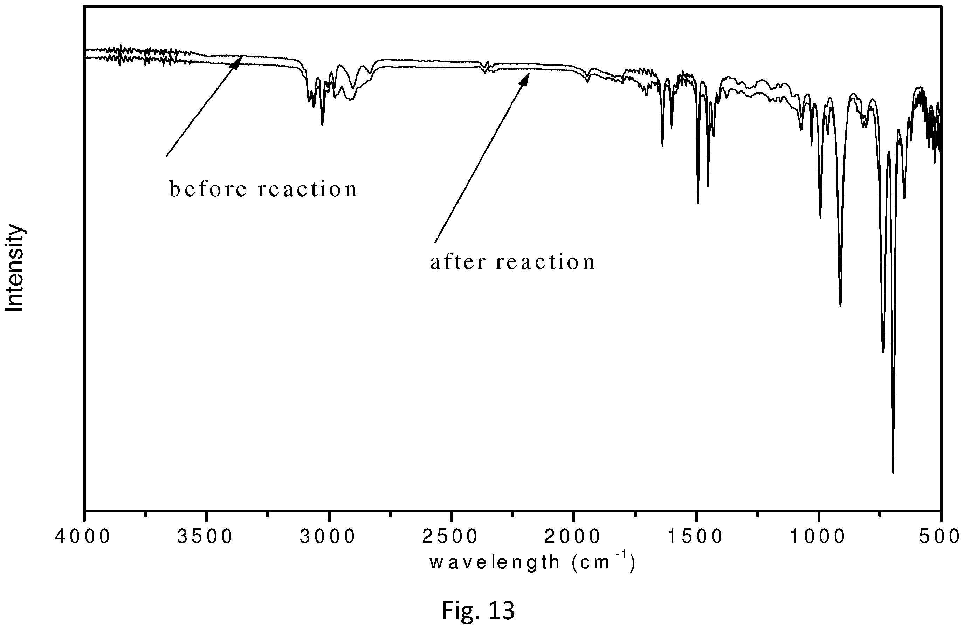

4.0 grams of a tungsten-phosphoric acid catalyst (H₃PW₁₂O₄₀, prepared in Example 1) were added to a quartz test tube equipped with a magnetic stirrer, maintained at a temperature of 80° C. 40mL of allylbenzene (available from Sigma-Aldrich) were also added to the test tube equipped with a magnetic stirrer, maintained at a temperature of 80°C. The contents were mixed continuously, and then irradiated with a visible 300W Xenon lamp. Each series of samples, withdrawn after 30min, 60min, 90min, 120min, 150min, 180min, 210min, 240min, 270min and 300mins, were analyzed via gas chromatography-mass spectrometry (GC-MS) and IR. The analysis showed that allylbenzene was isomerized trans-β-methyl styrene (seen in Figure 9-13).

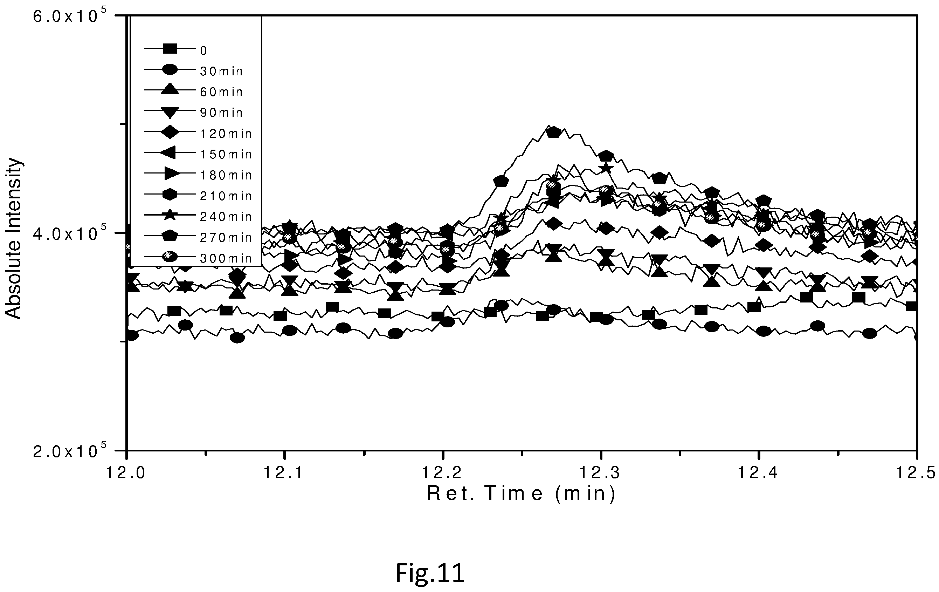

The assignment of the peaks in each of the IR spectra shown in Figure 11 are given below in Tables 9 and 10.

**Table 9 - Position of the IR bands (cm⁻¹) and assignment of allylbenzene (i.e. before exposure to reaction consitions)**

| **Wavenumber/cm⁻¹** | **Assignment** | **Ring modes Wilson notation** | **Propenyl modes** |
|---|---|---|---|
| 526cm⁻¹ | | | |
| 555cm⁻¹ | | | |
| 623cm⁻¹ | | | |
| 651cm⁻¹ | CH out-of-plane bending | | CH₂=twisting |
| 696cm⁻¹ | CH out-of-plane bending | | |
| 736cm⁻¹ | CH out-of-plane bending | | |
| 804cm⁻¹ | - | - | - |
| 819cm⁻¹ | - | - | - |
| 912cm⁻¹ | CH= out-of-plane bending (vinylic methylene group) | | CH₂= wagging |
| 964cm⁻¹ | CH out-of-plane bending | | |
| 993cm⁻¹ | C-Cstr./CHout-of-planebending | | CH₂=CH-out-of-plane bending |
| 1029 cm⁻¹ | C-C str. | | |
| 1072cm⁻¹ | C-C str. (CH in-plane bend.) | | CH₂= rocking |
| 1411cm⁻¹ | CH in-plane bending | | CH₂=scissoringconformer |
| 1431cm⁻¹ | CH in-plane bending | | CH₂= scissoring |
| 1452cm⁻¹ | C-C str./CH in-plane bend | | -CH₂- scissoring |
| 1494cm⁻¹ | C-C str./CH in-plane bend | | |
| 1602cm⁻¹ | C-C str./CH in-plane bend | | |
| 1637cm⁻¹ | C=C str. | | C=C str. |
| 1803cm⁻¹ | overtone | overtone | overtoneCH₂- wagging |
| 1944cm⁻¹ | combination | | |
| 2833cm⁻¹ broad | overtone (Fermi resonance) | | overtone -CH₂- scissoring |
| 2900cm⁻¹ broad | CH str. (Fermi resonance) | | -CH₂-sym. str. |
| 2978cm⁻¹ | CH str. | | -CH₂- asym. str. |
| 3005cm⁻¹ | CH str. | | CH₂= sym. str. |
| 3028cm⁻¹ | CH str. | | CH= str. |
| 3062cm⁻¹ | CH str. | | |
| 3082cm⁻¹ | CH str. | | |

**Table 10 - Position of the IR bands (cm-1) and assignment of the species arising from allylbenzene (i.e. after exposure to reaction conditions)**

| **Wavenumber/cm⁻¹** | **Assignment** | **Ring modes Wilson notation** | **Propenyl modes** |
|---|---|---|---|
| 623cm⁻¹ | | | |
| 651cm⁻¹ | CH out-of-plane bending | | CH₂=twisting |
| 696cm⁻¹ | CH out-of-plane bending | | |
| 736cm⁻¹ | CH out-of-plane bending | | |
| 804cm⁻¹ | - | - | - |
| 819cm⁻¹ | - | - | - |
| 912cm⁻¹ | CH= out-of-plane bending (vinylic methylene group) | More weak | CH₂= wagging |
| 964cm⁻¹ | CH out-of-plane bending | More strong | |
| 993cm⁻¹ | C-Cstr./CHout-of-planebending | More weak | CH₂=CH-out-of-plane bending |
| 1029 cm⁻¹ | C-C str. | | |
| 1072cm⁻¹ | C-C str. (CH in-plane bend.) | | CH₂= rocking |
| 1377cm⁻¹ | | | |
| 1411cm⁻¹ | CH in-plane bending | | CH₂=scissoringconformer |
| 1431cm⁻¹ | CH in-plane bending | | CH₂= scissoring |
| 1452cm⁻¹ | C-C str./CH in-plane bend | | -CH₂- scissoring |
| 1494cm⁻¹ | C-C str./CH in-plane bend | | |
| 1602cm⁻¹ | C-C str./CH in-plane bend | | |
| 1637cm⁻¹ | C=C str. | | C=C str. |
| 1654cm⁻¹ | | | |
| 1662cm⁻¹ | | | |
| 1670cm⁻¹ | | | |
| 1685cm⁻¹ | | | |
| 1705cm⁻¹ | | | |
| 1803cm⁻¹ | overtone | overtone | overtoneCH₂- wagging |
| 1944cm⁻¹ | combination | | |
| 2833cm⁻¹ broad | overtone (Fermi resonance) | | overtone -CH₂- scissoring |
| 2900cm⁻¹ broad | CH str. (Fermi resonance) | | -CH₂-sym. str. |
| 2978cm⁻¹ | CH str. | | -CH₂- asym. str. |
| 3005cm⁻¹ | CH str. | | CH₂= sym. str. |
| 3028cm⁻¹ | CH str. | | CH= str. |
| 3062cm⁻¹ | CH str. | | |
| 3082cm⁻¹ | CH str. | | |

While specific embodiments of the invention have been described herein for the purpose of reference and illustration, various modifications will be apparent to a person skilled in the art without departing from the scope of the invention as defined by the appended claims.

## Claims

1. A process for the migration of a carbon-carbon double bond within an unsaturated organic compound having 4 or more carbon atoms, the process comprising the step of:
a) contacting the unsaturated organic compound with a heteropoly acid;
wherein step a) is conducted in the presence of electromagnetic radiation having a
wavelength of less than or equal to 700 nm, and wherein the heteropoly acid is phosphotungstic acid.

2. The process of claim 1, wherein the unsaturated organic compound has 60 or fewer carbon atoms; optionally wherein the unsaturated organic compound has 30 or fewer carbon atoms; further optionally wherein the unsaturated organic compound has 20 or fewer carbon atoms.

3. The process of claim 1 or claim 2, wherein the unsaturated organic compound is a straight chain alkene or a branched alkene.

4. The process of any preceding claim, wherein step a) is conducted in the presence of electromagnetic radiation having a wavelength of 10-700 nm.

5. The process of any preceding claim, wherein step a) is conducted in the presence of electromagnetic radiation having a wavelength of 200-700 nm.

6. The process of any preceding claim, wherein step a) is conducted in the presence of electromagnetic radiation having a wavelength of 300-700 nm.

7. The process of any preceding claim, wherein the heteropoly acid has a Keggin structure.

8. The process of any preceding claim, wherein the heteropoly acid is provided on a support.

9. The process of claim 8, wherein the support is Al₂O₃.

10. The process of claim 8 or 9, wherein the support constitutes 0.1-90% by weight of the total weight of the heteropoly acid.

11. The process of claim 10, wherein the support constitutes 0.1-30% by weight of the total weight of the heteropoly acid.

12. The process of claim 10, wherein the support constitutes 15-25% by weight of the total weight of the heteropoly acid.

13. The process of any preceding claim, wherein the heteropoly acid has a BET surface area of ≥3 m²/g.

14. The process of any preceding claim, wherein the heteropoly acid has a pore volume of 0.1-1.0 ml/g.

15. The process of any preceding claim, wherein the heteropoly acid has a density of 0.1-20 g/ml.

## Patentansprüche

1. Verfahren zur Migration einer Kohlenstoff-Kohlenstoff-Doppelbindung innerhalb einer ungesättigten organischen Verbindung, die 4 oder mehr Kohlenstoffatome aufweist, wobei das Verfahren den folgenden Schritt umfasst:
a) Inkontaktbringen der ungesättigten organischen Verbindung mit einer Heteropolysäure;
wobei Schritt a) in der Gegenwart elektromagnetischer Strahlung mit einer Wellenlänge von weniger als oder gleich 700 nm durchgeführt wird,
und wobei die Heteropolysäure Phosphorwolframsäure ist.

2. Verfahren nach Anspruch 1, wobei die ungesättigte organische Verbindung 60 oder weniger Kohlenstoffatome aufweist; wahlweise, wobei die ungesättigte organische Verbindung 30 oder weniger Kohlenstoffatome aufweist; ferner wahlweise, wobei die ungesättigte organische Verbindung 20 oder weniger Kohlenstoffatome aufweist.

3. Verfahren nach Anspruch 1 oder Anspruch 2, wobei die ungesättigte organische Verbindung ein geradkettiges Alken oder ein verzweigtes Alken ist.

4. Verfahren nach einem vorhergehenden Anspruch, wobei Schritt a) in der Gegenwart elektromagnetischer Strahlung mit einer Wellenlänge von 10-700 nm durchgeführt wird.

5. Verfahren nach einem vorhergehenden Anspruch, wobei Schritt a) in der Gegenwart elektromagnetischer Strahlung mit einer Wellenlänge von 200-700 nm durchgeführt wird.

6. Verfahren nach einem vorhergehenden Anspruch, wobei Schritt a) in der Gegenwart elektromagnetischer Strahlung mit einer Wellenlänge von 300-700 nm durchgeführt wird.

7. Verfahren nach einem vorhergehenden Anspruch, wobei die Heteropolysäure eine Keggin-Struktur aufweist.

8. Verfahren nach einem vorhergehenden Anspruch, wobei die Heteropolysäure auf einem Träger bereitgestellt wird.

9. Verfahren nach Anspruch 8, wobei der Träger Al₂O₃ ist.

10. Verfahren nach Anspruch 8 oder 9, wobei der Träger 0,1-90 Gewichts-% des Gesamtgewichts der Heteropolysäure darstellt.

11. Verfahren nach Anspruch 10, wobei der Träger 0,1-30 Gewichts-% des Gesamtgewichts der Heteropolysäure darstellt.

12. Verfahren nach Anspruch 10, wobei der Träger 15-25 Gewichts-% des Gesamtgewichts der Heteropolysäure darstellt.

13. Verfahren nach einem vorhergehenden Anspruch, wobei die Heteropolysäure eine BET-Oberfläche von ≥3 m²/g aufweist.

14. Verfahren nach einem vorhergehenden Anspruch, wobei die Heteropolysäure ein Porenvolumen von 0,1-1,0 ml/g aufweist.

15. Verfahren nach einem vorhergehenden Anspruch, wobei die Heteropolysäure eine Dichte von 0,1-20 g/ml aufweist.

## Revendications

1. Procédé de migration d'une double liaison carbone-carbone à l'intérieur d'un composé organique insaturé ayant 4 atomes de carbone ou plus, le procédé comprenant les étapes de :
a) mise en contact du composé organique insaturé avec un hétéropolyacide ; dans lequel l'étape a) est réalisée en présence d'un rayonnement électromagnétique ayant une longueur d'onde inférieure ou égale à 700 nm, et dans lequel l'hétéropolyacide est l'acide phosphotungstique.

2. Procédé selon la revendication 1, dans lequel le composé organique insaturé a 60 atomes de carbone ou moins ; dans lequel le composé organique insaturé a éventuellement 30 atomes de carbone ou moins ; dans lequel le composé organique insaturé a en outre éventuellement 20 atomes de carbone ou moins.

3. Procédé selon la revendication 1 ou la revendication 2, dans lequel le composé organique insaturé est un alcène à chaîne linéaire ou un alcène ramifié.

4. Procédé selon l'une quelconque des revendications précédentes, dans lequel l'étape a) est réalisée en présence d'un rayonnement électromagnétique ayant une longueur d'onde de 10-700 nm.

5. Procédé selon l'une quelconque des revendications précédentes, dans lequel l'étape a) est réalisée en présence d'un rayonnement électromagnétique ayant une longueur d'onde de 200-700 nm.

6. Procédé selon l'une quelconque des revendications précédentes, dans lequel l'étape a) est réalisée en présence d'un rayonnement électromagnétique ayant une longueur d'onde de 300-700 nm.

7. Procédé selon l'une quelconque des revendications précédentes, dans lequel l'hétéropolyacide a une structure de Keggin.

8. Procédé selon l'une quelconque des revendications précédentes, dans lequel l'hétéropolyacide est monté sur un support.

9. Procédé selon la revendication 8, dans lequel le support est Al₂O₃.

10. Procédé selon la revendication 8 ou la revendication 9, dans lequel le support représente 0,1-90 % en poids du poids total de l'hétéropolyacide.

11. Procédé selon la revendication 10, dans lequel le support représente 0,1-30 % en poids du poids total de l'hétéropolyacide.

12. Procédé selon la revendication 10, dans lequel le support représente 15-25 % en poids du poids total de l'hétéropolyacide.

13. Procédé selon l'une quelconque des revendications précédentes, dans lequel l'hétéropolyacide a une surface BET de ≥3 m²/g.

14. Procédé selon l'une quelconque des revendications précédentes, dans lequel l'hétéropolyacide a un volume poreux de 0,1-1,0 mL/g.

15. Procédé selon l'une quelconque des revendications précédentes, dans lequel l'hétéropolyacide a une densité de 0,1- 20 g/mL.
